Europäisches Patentamt

European Patent Office

Office européen des brevets

(19)

(11) Publication number: **0 307 184**
**A2**

# EUROPEAN PATENT APPLICATION

(12)

(21) Application number: **88308270.3**

(22) Date of filing: **07.09.88**

(51) Int. Cl.⁴: **C 07 C 135/02**

(30) Priority: **08.09.87 US 94150    21.04.88 US 184504**

(43) Date of publication of application:
**15.03.89    Bulletin    89/11**

(84) Designated Contracting States:
**AT BE CH DE ES FR GB GR IT LI LU NL SE**

(71) Applicant: **ETHYL CORPORATION**
**451 Florida Boulevard**
**Baton Rouge, LA 70801 (US)**

(72) Inventor: **Bauer, Dennis Paul**
**11904 Tower Oaks Drive**
**Baton Rouge, LA 70810 (US)**

**Willard, Teresa Summerford**
**4159 Treuil Road**
**Port Allen, LA 70767 (US)**

**Laurenzo, Kathleen Susan**
**6212 Stumberg Lane, Apt. 209**
**Baton Rouge, LA 70816 (US)**

**Masterton, Frederick Richard**
**13215 Country Park Avenue**
**Baton Rouge, LA 70816 (US)**

(74) Representative: **Bizley, Richard Edward et al**
**BOULT, WADE & TENNANT 27 Furnival Street**
**London EC4A 1PQ (GB)**

(54) **Preparation of Amine Oxides.**

(57)    Tertiary amine oxides are made which are substantially free of nitrosamine impurities by reacting the desired tert-amine with aqueous hydrogen peroxide in the presence of carbon dioxide and at a temperature below about 45°C, especially below 40°C. Nitrosamines are suspect carcinogens and mutagens.

EP 0 307 184 A2

**Description**

# PREPARATION OF AMINE OXIDES

It is known to make tert-amine oxides by the reaction of aqueous hydrogen peroxides with tert-amines. This reaction can also be conducted using a co-solvent such as a lower alcohol to prevent gel formation.

Murato U. S. 4,247,480 discloses that the formation of amine oxides from tert-amine by reaction with hydrogen peroxide can be promoted by including 0.01-2 weight percent carbon dioxide based on the tert-amine. Murato iu demonstrates the process at 55-65°C and obtains excellent yields of amine oxides.

It is known that nitrosamine impurities are routinely formed when reacting hydrogen peroxide with tert-amines. This would not be a problem if it were not for the fact that nitrosamines are suspected carcinogens and mutagens and even small amounts cannot be tolerated in products intended for contact with humans, "Nitrosamines: Assessing the Relative Risk" Chemical & Engineering News, pages 20-26, March 31, 1980. The major uses for tert-amine oxides are in cosmetic and surfactant formulations including such things as laundry detergents, hair shampoo and hair conditioners, all of which come in contact with humans. This necessitates special procedures to remove nitrosamine impurities from tert-amine oxides before they can be used in their major marketing areas. Such treatments include the exposure of the tert-amine oxide to ultra-violet irradiation. Therefore a need exists for a process for making tert-amine oxides that are substantially free of nitrosamine impurities without resorting to any special purification procedure after the synthesis operation.

It has been discovered that tert-amine oxides that are substantially free of nitrosamine impurities can be made by reacting the desired tert-amines with aqueous hydrogen peroxide in the presence of a promoter formed from carbon dioxide if the reaction is conducted at a temperature of 45°C or lower and preferably below 40°C.

A preferred embodiment of the invention is a process for oxidizing a tert-amine by reaction with hydrogen peroxide to form a tert-amine oxide, said process comprising contacting said tertamine with aqueous hydrogen peroxide in the presence of a promoter formed by adding carbon dioxide to the reaction mixture, said process being further characterized by being conducted at a temperature below about 45°C.

In a more preferred emlodiment the tert-amine has the formula $R^1R^2R^3N$ wherein $R^1$ is an alkyl group containing 1-30 carbon atoms and $R^2$ and $R^3$ are alkyl groups containing 1-30 carbon atoms, cycloalkyl groups containing 5-12 carbon atoms, aryl groups containing 6-12 carbon atoms, aralkyl groups containing 7-12 carbon atoms or any two of the R groups can join to form a carbocyclic or heterocyclic ring or all three of the R groups may participate to form a pyridine ring, said process being conducted at a temperature in the range of 0-45°C whereby the formation of nitrosamine impurities is substantially inhibited.

The process is appiicable to any of a broad range of tert-amines such as butyldimethylamine, hexyldimethylamine, isobutyldimethylamine, 2-ethylhexyldimethylamine, octyldimethylamine, decyldimethylamine, dodecyldimethylamine, tetradecyldimethylamine, hexadecyldimethylamine, eicosyldimethylamine, docosyldimethylamine, triacontyldimethylamine, tributylamine, butyldiethylamine, isobutyldiethylamine, decylbutylethylamine, hexadecylhexylmethylamine, eicosyldibutylamine, trioctylamine, tridodecylamine, dieicosylethylamine, ditriacontylmethylamine, N,N-dimethyl-aniline, N-methyl-N-dodecylaniline, cyclopentyldimethylamine, cyclohexyldimethylamine, dicyclohexylmethylamine, cyclododecyldimethylamine, diphenylbutylamine, para-tolyldiethylamine, α-naphthylbutylmethylamine, benzylbutylmethylamine, α-methylbenzylbutylmethylamine, (4-butylbenzyl)octylmethylamine, dibenzylbutylamine, (4-pentyl)benzyldibutylamine, N-butylmorpholine, N-methylmorpholine, N-methylpiperidine, N-dodecylpiperidine, N-octadecylpiperidine, N-triacontylpiperidine, N-methylpiperazine, N-butylpiperazine, N-octylpiperazine, N-phenylpiperidine, N-benzylpiperidine, N-cyclohexylpiperidine, and pyridine.

In a more preferred embodiment the tert-amine is a primary trialkylamine having the structure $R^1R^2R^3N$ wherein $R^1$, $R^2$ and $R^3$ are primary alkyls having 1-30 carbon atoms. Representative examples of these include but are not limited to trimethylamine, tri-n-pentylamine, tri-n-dodecylamine, n-octadecyl di-(n-butyl)amine, n-eicosyl di-(n-decyl)amine, n-triacontyl, and n-dodecyl methylamine.

In a still more preferred embodiment $R^1$ is a primary alkyl group containing 6-20 carbon atoms and $R^2$ and $R^3$ are independently selected from methyl and ethyl groups.

In a further preferred embodiment $R^1$ is a mainly linear primary alkyl containing 8-20 carbon atoms and $R^2$ and $R^3$ are methyl groups. By "mainly linear" is meant that over 50 percent, more preferably 70 percent and most preferably 90 percent of the $R^1$ groups are linear alkyls containing 8-20 carbon atoms.

Examples of these preferred embodiments are octyldimethylamine, decyldimethylamine, dodecyldimethylamine, tetradecyldimethylamine, hexadecyldimethylamine, octadecyldimethylamine, eicosyldimethylamine and mixtures thereof.

In another more preferred embodiment of the invention, both $R^1$ and $R^2$ are independently selected from primary alkyls containing 6-20 carbon atoms and $R^3$ is a methyl or ethyl group.

In a highly preferred embodiment $R^1$ and $R^2$ are independently selected from mainly linear primary alkyl groups containing 8-20 carbon atoms and $R^3$ is methyl. Examples of this highly preferred embodiment are dioctylmethylamine, didecylmethylamine, didodecylmethylamine, ditetradecylmethylamine, dihexadecylmethylamine, dioctadecylmethylamine, dieicosylmethylamine, decyloctylmethylamine, dodecyloctylmethylamine, tetradecyldecylmethylamine, hexadecyltetradecylmethylamine, octadecylhexadecylmethylamine, eicosyldodecylmethylamine, and mixtures thereof.

Any aqueous hydrogen peroxide can be used including those containing 3-100 percent $H_2O_2$. Preferably the hydrogen peroxide is 20-70 weight percent active $H_2O_2$. When the tertamine is linear $C_{8-20}$ alkyl dimethylamine, it is preferred that the aqueous hydrogen peroxide be 20-40 weight percent $H_2O_2$ to avoid gel formation. Alternatively, more concentrated hydrogen peroxide can be used and additional water either added initially or co-fed to maintain a stirrable reaction mixture. In this mode of operation it is the "net" $H_2O_2$ concentration, taking into account the additional water, that controls gel and should be kept in a gel-free range. For example, co-feeding 100 g of 50 percent aqueous hydrogen peroxide and 100 g of water gives a "net" 25 percent aqueous hydrogen peroxide. Likewise prior addition of 100 g of water to the tert-amine followed by feeding 100 grams of 50 percent aqueous hydrogen peroxide gives a "net" 25 percent aqueous hydrogen peroxide. Likewise, co-solvents such as lower alcohol, e.g., isopropanol, isobutanol and the like, can be used to avoid gelation.

The amount of hydrogen peroxide should be at least a stoichiometric amount. A useful range is 1-5 moles of $H_2O_2$ and more preferably 1-1.5 moles of $H_2O_2$ per mole of tert-amine. A highly preferred amount is 1.0-1.3 moles of $H_2O_2$ and especially 1.05-1.2 moles of $H_2O_2$ per mole of tert-amine. Any substantial amount of unreacted $H_2O_2$ remaining after the reaction can be destroyed by the addition of a reducing agent or a peroxide decomposition catalyst.

When the process is conducted using a di-linear alkyl methylamine, the process can be carried out using more concentrated aqueous hydrogen peroxide such as 45-70 weight percent hydrogen peroxide. When the di-linear alkyls contain 6-12 carbon atoms each, the reaction mixture will remain substantially gel free. When the di-linear alkyls contain 14 or more carbon atoms the reaction mixture will set up to a dry flakeable solid on cooling.

The reaction must be conducted using added carbon dioxide. The reaction can be pressurized (e.g., to 0.03-3 MPa or higher) with carbon dioxide or merely provided with a carbon dioxide blanket. An effective way to conduct the reaction is to maintain a carbon dioxide sweep over the liquid reaction phase during all or a portion of the reaction. If the reaction rate slows down towards the end of the reaction additional carbon dioxide can be injected.

The amount of carbon dioxide added should be an amount which catalyzes the reaction causing it to proceed at a faster rate. This can range from 0.005 weight percent up to the solubility limit of the carbon dioxide in the tert-amine. When oxidizing a $C_{6-20}$ alkyl dimethylamine it was found that an increase in catalytic effect can be achieved by using large amounts of carbon dioxide. This is especially important when operating at low temperatures, e.g. 20-39°C, where the increase in carbon dioxide permits the reaction to go to completion in about 3 hours or less. Thus, an especially preferred embodiment of the invention is a process of reacting a primary $C_{6-20}$ alkyl dimethylamine with aqueous hydrogen peroxide at a temperature below 40°C and in the presence of at least 3 weight percent up to the limit of solubility of carbon dioxide based on the weight of the primary $C_{6-20}$ alkyl dimethylamine in the reaction mixture. For example, 3 to 7 weight percent carbon dioxide can be dissolved in such amines at around ambient temperature, e.g. 20-35°C. Exceptionally fast reaction rates have been achieved at 3-5 wt percent carbon dioxide. This is shown in the later examples.

The oxidation can be conducted at any temperature high enough to cause the desired reaction to proceed but not so high as to cause excessive decomposition of the reactants or products. In general a useful reaction temperature if nitrosamines are not a concern is 30-140°C. However, in order to inhibit the formation of nitrosamines, the reaction must be conducted at 45°C or lower, more preferably 40°C or lower and most preferably below 30°C. In order to operate at this low temperature at a reasonable reaction rate, it is necessary to utilize the carbon dioxide promoter. Thus, the invention resides not only in the low reaction temperature, but also in the combination of the low reaction temperature with the use of the carbon dioxide promoter.

A useful reaction temperature for minimizing nitrosamine formation is 0-45°C and a more preferred reaction temperature is 20-40°C. Lower nitrosamine levels are achieved if the process is conducted at a temperature below 40°C. Thus, a highly preferred temperature in which to operate is 20-39°C and especially 25-35°C and more preferably 25-30°C. Excellent results have been achieved around 25°C.

The reaction time is a variable and depends on the tert-amine selected, the reaction temperature and the reaction scale. Generally, the reaction is complete in 1-24 hours, more often in 2-12 hours and most often in 3-8 hours. Progress of the reaction after all of the hydrogen peroxide and tert-amine have been mixed can be followed by withdrawing small samples and analyzing for free amine. The reaction is considered complete when the free amine level drops below about 3 weight percent. At this stage the product may be packaged for use or shipment. Any residual free amine will continue to decrease on standing and/or during packaging or storage prior to shipment until it finally becomes non-detectable.

The substantial elimination of the nitrosamine impurities achieved with the present improved process compared to the results achieved at higher temperatures is shown in the following comparative examples.

Example 1

This example was not conducted under the reaction conditions of the present invention.

In a reaction flask fitted with a stirrer, thermometer and addition funnel was placed 250 grams of dodecyldimethylamine. While stirring at 65°C, and sweeping carbon dioxide over the liquid phase, 86 grams (1.1 equivalent) of 50 weight percent aqueous hydrogen peroxide was added over a 1-hour period together with 584 ml of water which is equivalent to using 670 g of 6.4 weight percent aqueous hydrogen peroxide. This calculates to give a 30 weight percent aqueous amine oxide solution at the end of the reaction. Stirring was continued at 65°C for two additional hours. The product was cooled and analyzed for dodecyldimethylamine oxide, unreacted amine and nitrosamine. Conversion to dodecyldimethylamine oxide was substantially complete. Analysis by high pressure liquid chromatography using a Thermal Energy Analyzer (by Thermedics Incorporated) was 96 ppb N,N-dimethylnitrosamine (limit of detection 20 ppb).

## Example 2

This example was carried out following the present improved invention.

The reaction setup was the same as in Example 1. The reactants were also the same. The only difference was that this example was conducted at 45°C and the reaction was conducted over a period of 4 hours. Conversion to dodecyldimethylamine oxide was essentially complete. The product was analyzed for nitrosamine using the same HPLC Thermal Energy Analyzer as used in Example 1.

## Examples 3 - 5

The procedure of Example 2 was repeated at 35°C and 38°C. Reaction condition and results for all runs is shown in the following table which includes a commercial dodecyldimethylamine oxide for comparison.

Table I

| Example | Reaction Conditions | | | Product Composition | | | |
| | Temp. | Time | $CO_2$ | Wt. Percent | | Nitrosamine(ppb) | |
| | | | | Amine Oxide | Residual Amine | A[1] | B[2] |
| 1 | 65°C | 3 hrs. | Yes | 28.9 | 0.4 | 96 | N.D. |
| 2 | 45°C | 4 hrs. | Yes | 28.2 | 0.89 | 16 | N.D. |
| 3 | 38°C | 3 hrs. | Yes[3] | 29.7 | 0.1 | N.D. | N.D. |
| 4 | 35°C | 5 hrs. | Yes | 28.5 | 0.47 | N.D. | N.D. |
| 5 | 35°C | 6 hrs. | Yes | 30.2 | 0.03 | N.D. | N.D. |
| 6[4] | 65°C | - | - | - | - | 570 | 230 |

A. N,N-dimethylnitrosamine.
B. N-methyl-N-dodecylnitrosamine.

1. Detection limit 20 ppb for Example 1, 2, 4 and 5; 10 ppb in Example 3.
2. Detection limit 100 ppb in Examples 1, 2, 3 and 5; 80 ppb in Example 3.
3. 0.9 wt % $CO_2$ was initially dissolved in the tert-amine without use of $CO_2$ sweep.
4. Commercial dodecyldimethylamine oxide.

As explained previously, the use of large amounts of carbon dioxide permits the process to be conducted at a fast reaction rate even at temperatures below 40°C, e.g. 20-39°C, and especially around 25°C thereby achieving nitrosamine levels below the limit of detection by present analytical methods. These results are shown in the following Table II.

Table II

| Example | Reaction Conditions | | | Product Composition | | | |
| | Temp. | Time | $CO_2$ | Wt. Percent | | Nitrosamine(ppb) | |
| | | | | Amine Oxide | Residual Amine | A[3] | B[3] |
| 7 | 25°C | 4.5 hrs[1] | 3.5% | 29.8 | 0.04 | N.D. | N.D. |
| 8 | 25°C | 4.25 hrs[2] | 5% | 28.5 | 0.09 | N.D. | N.D. |

1. Reaction was 95% complete in 4.5 hours and went to completion on standing overnight at room temperature.
2. Reaction was 96% complete in 4.25 hours and went to completion on standing overnight at room temperature.
3. Detection limits 10 ppb N,N-dimethylnitrosamine and 80 ppb N-methyl-N-dodecylnitrosamine.

These results show that the reaction conducted at 65°C gave a product with a low (96 ppb) but still detrimental amount of nitrosamine. When the same reaction was conducted at 45°C, nitrosamines dropped sharply and at below 40°C, e.g. 38°C, nitrosamines usually could not be detected. When conducted at 25°C, nitrosamines have never been detected following the present process. The invention therefore fulfills a long-felt need and provides a health and environment benefit.

In a still further embodiment it has been discovered that it is not necessary to conduct the reaction in its entirety below 40°C. The reaction can be initiated at a low temperature by starting feed of the aqueous hydrogen peroxide to the amine in the presence of added carbon dioxide and then allowing the temperature to gradually rise from the heat of reaction. In this embodiment the reaction is preferably started at 30°C or lower, e.g., 25°C, and permitted to rise in temperature as the hydrogen peroxide is added. Some cooling may be required to limit the rate of temperature rise. The rate of temperature increase should be such that it is still below 40°C until about 20 weight percent of the stoichiometric amount of aqueous hydrogen peroxide has been fed. More preferably the reaction should be held below 40°C until at least 25 weight percent and most preferably at least 30 weight percent of the stoichiometric amount of hydrogen peroxide has been added. Following this the temperature can rise to whatever the heat of reaction takes it during the remainder of the aqueous hydrogen peroxide feed, preferably still less than 70°C. The mixture can then be allowed to cool down to ambient or cooling can be applied to hasten the temperature drop to ambient. The following example shows this embodiment of the invention.

## Example 9

In a reaction vessel was placed 250 g (1.17 moles) of n-dodecyl dimethylamine and 563 g of water. Carbon dioxide was injected into the mixture until a 2.5 g weight gain is attained (1.0 weight percent of the tert-amine). While stirring at 25°C, 50 weight percent aqueous hydrogen peroxide feed was commenced without cooling. Temperature rise continued as the hydrogen peroxide was added as follows:

| Reaction Time (min) | 50% Hydrogen Peroxide Feed (g) | Temperature (°C) |
|---|---|---|
| Start | - | 27 |
| 15 | 13.61 | 33 |
| 30 | 22.87 | 38 |
| 45 | 32.85 | 43 |
| 60 | 44.99 | 48 |
| 90 | 66.79 | 52 |
| 105 | 83.79 | 51 |
| 120 | - | 60 |
| 180 | - | 65 |
| 240 | - | 65 |

Upon completion of hydrogen peroxide feed (105 min), heat was applied to raise the temperature to 65°C.

After 4 hours from the start of the reaction, the tert-amine conversion was over 99 percent. The mixture was then permitted to cool to ambient. Nitrosamine was below the level of detection of 10 ppb N,N-dimethyl nitrosamine and 80 ppb N-methyl-N-dodecyl nitrosamine.

## Example 10

This process was conducted in the same general procedure as Example 9 except that 3 wt % carbon dioxide based on amine was added. The hydrogen peroxide feed was on the following time-temperature schedule.

| Reaction Time (min) | 50% Hydrogen Peroxide Feed (g) | Temperature (°C) |
|---|---|---|
| 0 | Start | 28 |
| 15 | 12.15 | 34 |
| 30 | 25.76 | 41 |
| 45 | 37.27 | 46 |
| 60 | 52.05 | 52 |
| 90 | 72.57 | 51 |
| 105 | 83.88 | 50 |
| 120 | - | 60 |
| 180 | - | 65 |

After complete addition of peroxide, the mixture was heated to 60-65°C.

After a 3 hour total time from start, the tert-amine conversion was over 99 percent. The reaction mixture was permitted to cool to ambient. Nitrosamines were below the level of detection. In this graduated temperature embodiment the product is substantially nitrosamine-free by present detection methods or contains only low levels of nitrosamine, e.g. below 25 ppb. In contrast as shown in comparative Example 1, a reaction conducted in its entirety at 65°C gave a product containing 96 ppb N,N-dimethylnitrosamine.

**Claims**

1. A process for oxidizing a tert-amine by reaction with hydrogen preoxide to form a tert-amine oxide and comprising contacting said tert-amine with aqueous hydrogen peroxide in the presence of a promoter formed by adding carbon dioxide to the reaction mixture, characterized by being conducted at a temperature below about 45°C whereby the nitrosamine content of the tert-amine oxide product is substantially reduced compared to the nitrosamine content of tert-amine oxides produced by the same process at higher temperatures.

2. A process of Claim 1 conducted at a temperature below 40°C, preferably at a temperature of from 20-39°C.

3. A process of Claim 1 or Claim 2 wherein said tert-amine has the formula $R^1R^2R^3N$ wherein $R^1$ is an alkyl group containing 1-30 carbon atoms and $R^2$ and $R^3$ are alkyl groups containing 1-30 carbon atoms, cycloalkyl groups containing 5-12 carbon atoms, aryl groups containing 6-12 carbon atoms, aralkyl groups containing 7-12 carbon atoms or any two of the R groups can join to form a carbocyclic or heterocyclic ring or all three of the R groups may participate to form a pyridine ring, said process being conducted at a temperature in the range of 0-45°C whereby the formation of nitrosamine impurities is substantially inhibited.

4. A process of Claim 1 or Claim 2 wherein said tert-amine has the structure $R^1R^2R^3N$ and at least $R^1$ of $R^1$ and $R^2$ is selected from primary alkyl groups containing 6-20 carbon atoms and $R^3$ is methyl or ethyl, and , if not as defined above, $R^2$ is methyl or ethyl.

5. A process of Claim 4 wherein at least $R^1$ of $R^1$ and $R^2$ is selected from primary mainly linear alkyl groups containing 8-20 carbon atoms and $R^3$ is methyl, and if not as defined above, $R^2$ is methyl.

6. A process of Claim 4 or Claim 5 wherein at least $R^1$ of $R^1$ and $R^2$ is decyl, dodecyl, tetradecyl, hexadecyl, or octadecyl.

7. A process of any one of Claims 1 to 6 wherein the hydrogen peroxide is 20-70 weight percent active $H_2O_2$.

8. A process of any one of Claims 1 to 7 wherein the amount of carbon dioxide is from 3 to 7 weight percent based on the weight of said tert-amine.

9. A process of any one of Claims 1 to 8 carried out in the presence of a co-solvent, such as a lower alcohol.

10. The use of a process for oxidizing a tert-amine by reaction with hydrogen peroxide to form a tert-amine oxide and comprising contacting said tert-amine with aqueous hydrogen peroxide in the presence of a promoter formed by adding carbon dioxide to the reaction mixture, said process being conducted at a temperature below about 45°C, in the production of an ingredient for a cosmetic or surfactant formulation.